Europäisches Patentamt

European Patent Office

Office européen des brevets

(19)

(11) Veröffentlichungsnummer: **0 189 491**
**A1**

(12)                      # EUROPÄISCHE PATENTANMELDUNG

(21) Anmeldenummer: 85100793.0

(22) Anmeldetag: 26.01.85

(51) Int. Cl.⁴: **A 61 M 5/14**

(43) Veröffentlichungstag der Anmeldung:
06.08.86 Patentblatt 86/32

(84) Benannte Vertragsstaaten:
AT BE CH DE FR GB IT LI LU NL SE

(71) Anmelder: INTERMEDICAT GMBH
Gerliswilstrasse 74
CH-6020 Emmenbrücke(CH)

(72) Erfinder: Rosskopf, Gerhard, Dr.
Heiligenbergstrasse 29
D-3501 Fuldabrück-Dörnhagen(DE)

(72) Erfinder: Anis, Peter
Liegnitzer Strasse 5
D-3508 Meisungen(DE)

(74) Vertreter: von Kreisler, Alek, Dipl.-Chem. et al,
Deichmannhaus am Hauptbahnhof
D-5000 Köln 1(DE)

(54) Vorrichtung zur dosierten gleichzeitigen Infusion von Lösungen.

(57) Mehrere Infusionsbehälter (A1 bis A4) sind über Ablaufleitungen (B1 bis B4) mit einer Sammelleitung (12) verbunden, in die eine volumetrische Pumpe (13) eingesetzt ist, welche das Lösungsgemisch zum Patienten fördert. Einem Rechengerät (11) werden von Vorwahlschaltern (S1 bis S4) Infusionsraten zugeführt. Das Steuergerät (11) berechnet hieraus Tropfenzahlen und steuert Ventile (D1 bis D4) in den Ablaufleitungen so, daß jeweils ein Ventil geöffnet gehalten wird, bis in einer zugehörigen Tropfkammer (C1 bis C4) eine der Infusionsrate entsprechende Tropfenzahl gefallen ist.

EP 0 189 491 A1

./...

FIG. 1

Vorrichtung zur dosierten gleichzeitigen Infusion von Lösungen

Die Erfindung betrifft eine Vorrichtung zur dosierten gleichzeitigen Infusion von Lösungen aus mehreren Infusionsbehältern, mit Ablaufleitungen, die von den Behältern zu einer Sammelleitung führen und jeweils ein gesteuertes Ventil enthalten, einer in der Sammelleitung vorgesehenen Pumpe mit wählbarem Fördervolumen und mit einem Rechner, der die Ventile entsprechend eingestellten Infusionsraten der einzelnen Lösungen zeitlich nacheinander zyklisch öffnet.

Zur gleichzeitigen Zuleitung mehrerer Infusionslösungen zu einem Patienten ist es bekannt, in jede der Ablaufleitungen die von einem Lösungsbehälter zu einer Sammelleitung führen, ein Ventil einzuschalten und diese Ventile zeitlich nacheinander zu öffnen, so daß zu jedem Zeitpunkt nur eines der Ventile geöffnet ist (DE-OS 28 55 713). In die Sammelleitung ist eine volumetrische Pumpe eingesetzt, deren Fördervolumen der Summe der Infusionsraten sämtlicher Lösungen entsprechen kann. Die Pumpe fördert die Lösungen bzw. das Lösungsgemisch zum Patienten. In der Praxis werden als Infusionsbehälter, die die Lösungen enthalten, in der Regel Einmalbehälter in Form von Glasflaschen, Kunststofflaschen, PVC-Beuteln o.dgl. eingesetzt. Derartige Einmalbehälter haben kein Belüftungsfilter, so daß in den Behältern Unterdrücke oder auch Überdrücke auftreten können. Sofern ein Belüftungsfilter an dem in den Behälter hineinführenden Überleitungsgerät vorgesehen ist, besteht die Gefahr, daß das Filter mit der Flüssigkeit in Berührung kommt und von dieser benetzt wird, so daß es weitgehend luftundurchlässig wird. Nachteilige Folgen treten auch auf, wenn Lösungen mit unterschiedlichen Viskositäten benutzt werden. Da das bekannte Gerät eine reine Zeitsteuerung vornimmt, fließen Flüssigkeiten mit unterschiedlichen Viskositäten in unterschiedlichen Mengen pro Zeiteinheit zur Sammelleitung. Aus diesem Grunde kann das eingestellte Mischungsverhältnis nicht mit hinreichender Genauigkeit beibehalten werden. Ähnliches gilt auch für den Fall, daß die Schlauchleitungen unterschiedliche Strömungswiderstände haben. Dies kann z.B. dann der Fall sein, wenn einer der Schläuche abgeknickt ist oder wenn Verstopfungen im Schlauchsystem auftreten. Derartige Beeinflußungen der pro Zeiteinheit durch das Leitungssystem hindurchfließenden Flüssigkeitsmengen führen häufig dazu, daß dem Patienten nicht die richtigen Lösungsmengen verabreicht werden. Wenn entweder die Teilmengen oder die Gesamtmenge überwacht werden, treten häufig Alarmmeldungen auf, die auf das Unterschreiten oder Überschreiten eines Grenzwertes zurückzuführen sind und von dem Klinikpersonal nicht ohne weiteres definiert werden können.

Bekannt ist ferner eine Infusionseinrichtung (DE-A 27 30 736), bei der zwei Infusionsbehälter über Ablaufleitungen mit einer gemeinsamen Sammelleitung verbunden sind. Jede Ablaufleitung enthält eine Tropfkammer mit einem Tropfenzähler. Ein Steuergerät steuert Ventile, die in den Ablaufleitungen enthalten sind, so, daß jeweils zu vorgegebenen Zeitpunkten ein Ventil geöffnet wird. Wenn daraufhin in der Tropfkammer ein einziger Tropfen erzeugt worden ist, wird dies durch den zugehörigen Tropfendetektor erkannt und das Ventil wird wieder geschlossen. Wenn der eine Infusionsbehälter geleert worden ist oder wenn in der an diesen Infusionsbehälter angeschlossenen Ablaufleitung ein Strömungshindernis auftritt, wird das Ventil dieses Infusionsbehälters geschlossen und die Lösung aus dem zweiten Infusionsbehälter in derselben Weise entnommen. Diese bekannte Vorrichtung dient nicht zur gleichzeitigen Infusion mehrerer Lösungen, sondern sie überwacht die Infusion aus einem einzigen Infusionsbehälter und sieht einen Ersatz-Infusionsbehälter vor, dem bei Entleerung oder Ausfall des ersten Systems anschließend Infusionslösung entnommen wird.

Bei einer speziellen Variante können aus zwei Flüssigkeitsbehältern verschiedene Flüssigkeiten mit individuell vorwählbaren Frequenzen abwechselnd abgegeben werden. Jeweils nach Erreichen einer vorgegebenen Tropfenzahl aus einem Behälter nimmt eine Steuerschaltung die Umschaltung auf den anderen Behälter vor. Hierbei müssen die Tropfenzahlen unmittelbar voreingestellt werden. Es genügt nicht, lediglich die Infusionsraten bzw. die Mengenverhältnisse vorzuwählen.

Der Erfindung liegt die Aufgabe zugrunde, eine Vorrichtung der im Oberbegriff des Patentanspruchs 1 angegebenen Art dahingehend weiterzubilden, da´ die Genauigkeit der Mengendosierung sowohl hinsichtlich der Dosierung der einzelnen Infusionslösungen als auch hinsichtlich der Gesamtmenge erhöht wird, wobei am Gerät lediglich die Infusionsraten vorgewählt werden müssen.

Die Lösung dieser Aufgabe erfolgt mit den in dem kennzeichnenden Teil des Patentanspruchs 1 angegebenen Merkmalen.

Nach der Erfindung erfolgt die Zufuhr der einzelnen Teilmengen der Infusionslösungen nicht durch zeitabhängige Steuerung der Ventile, sondern mengenabhängig, wobei die Messung der Flüssigkeitsmenge durch Tropfenzählung erfolgt. Während bei zeitgesteuerten Infusionsraten die Meßungenauigkeiten in der Praxis größer sind als 25 %, wird bei Anwendung der erfindungsgemäßen Vorrichtung der Fehler durch die Abweichungen der Tropfengröße bestimmt. Diese sind kleiner als 10 %. Die vom Arzt zu wählenden Infusionsraten für die einzelnen Lösungen werden in dem Steuergerät in proportionale Tropfenzahlen umgerechnet. Jedes Ventil bleibt so lange geöffnet, bis die diesem Kanal zugeordnete Tropfenzahl erreicht ist. Danach wird das Ventil geschlossen und das Ventil des nächsten Kanals geöffnet. Es werden jeweils bestimmte Tropfenzahlen ermittelt und der Sammelleitung zugeführt, bis eine Umschaltung erfolgt. Die fest vorgegebene Mindesttropfenzahl ist jeweils die kleinste Einheit an Flüssigkeitsmenge, die der Sammelleitung in einem Intervall der Ventilsteuerung zugeführt wird. Auf diese Weise erhält man eine Bezugsbasis für die Berechnung der Tropfenzahlen.

Wenn sich aus den eingegebenen Infusionsraten ergibt, daß eine der Infusionslösungen mit einer Tropfenzahl zugeführt werden müßte, die keine ganze Zahl darstellt, kann man diese Tropfenzahl entweder aufrunden oder abrunden, weil innerhalb einer Öffnungsphase eines Ventils natürlich nur immer ganze Tropfen abgegeben werden. Ein solcher Auf- oder Abrundungsvorgang würde jedoch zu einer Ungenauigkeit bei der Einhaltung der Infusionsrate für die betreffende Lösung führen. Zur Vermeidung solcher Ungenauigkeiten ist gemäß einer vorteilhaften Weiterbildung der Erfindung eine Einrichtung vorgesehen, die aus den eingegebenen Infusionsraten proportionale Tropfenzahlen errechnet und im Falle einer nicht-ganzzahligen Tropfenzahl einer Lösung eine Abrundung auf die niedrigere ganze Zahl vornimmt, den sich ergebenden Rest speichert und den Rest bei dem nächstfolgenden Zyklus zu der nicht-ganzzahligen Tropfenzahl derselben Lösung hinzuaddiert. Auf diese Weise wird die Summe der über mehrere Zyklen akkumulierten Restwerte irgendwann größer als 1, so daß dann für diesen Kanal ein zusätzlicher Tropfen erzeugt wird. Dies bedeutet, daß für eine bestimmte Lösung beispielsweise in vier Zyklen jeweils acht Tropfen und in jedem fünften Zyklus neun Tropfen erzeugt werden.

Vorzugsweise ist die Pumpe mit einer der Summe der Infusionsraten der einzelnen Lösungen entsprechenden Förderrate betrieben. Die Pumpe läuft hierbei mit konstanter Förderrate und sie erzeugt in den Tropfkammern einen Unterdruck, wodurch Flüssigkeit aus dem betreffenden Lösungsbehälter angesaugt und die Tropfenbildung gefördert wird.

Eine Überwachung der Gesamt-Fördermenge, die von der Pumpe gefördert wird, kann dadurch erfolgen, daß in der Sammelleitung ein Durchflußmesser vorgesehen ist, der bei Abweichungen von der vorgesehenen Förderrate ein Alarmsignal auslöst. Dieser Durchflußmesser besteht vorzugsweise aus einer Tropfkammer mit Tropfenzähler. Etwaige Abweichungen von der vom Steuergerät berechneten Gesamt-Durchflußrate können auch dazu benutzt werden, die Steuersignale der Pumpe und/oder der Ventile, zu verändern.

Im folgenden werden unter Bezugnahme auf die Zeichnungen Ausführungsbeispiele der Erfindung näher erläutert.

Es zeigen:

Fig. 1    eine schematische Darstellung der Vorrichtung,

Fig. 2    eine vergrößerte Darstelung einer Tropfkammer mit Tropfendetektor,

Fig. 3    Flußdiagramme von Rechen- und Funktionsabläufen im Steuergerät und

Fig. 4    ein Blockschaltbild der wesentlichen Komponenten einer anderen Ausführungsform des Steuergerätes.

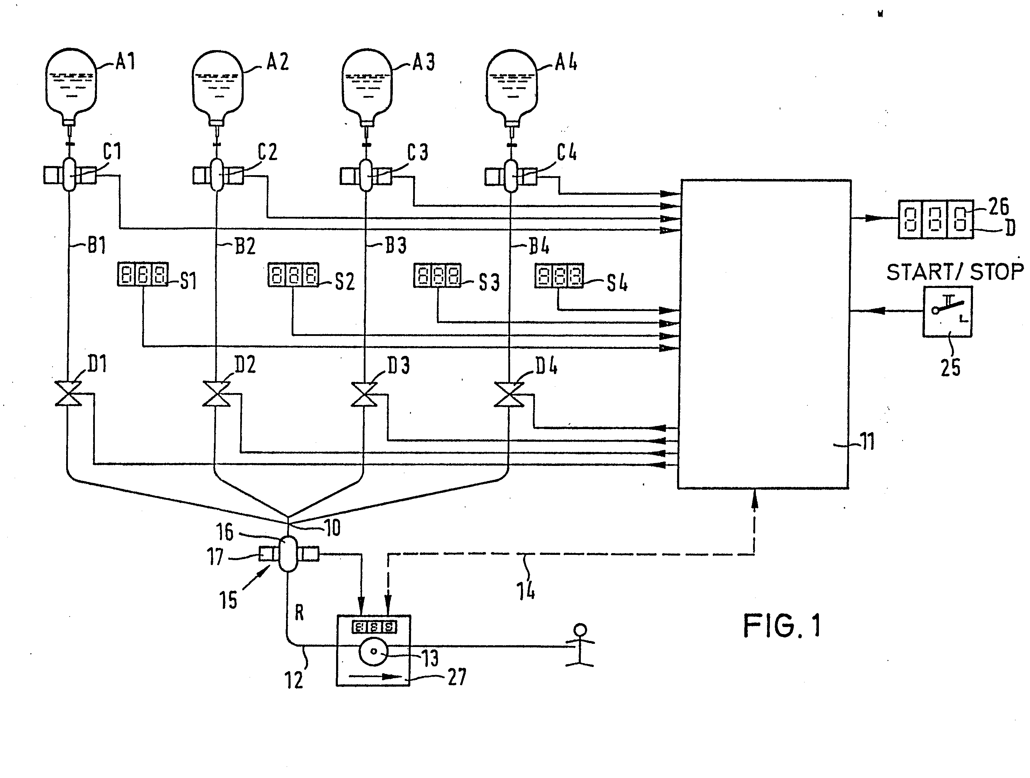

In Fig. 1 sind vier Infusionsbehälter A1,A2,A3,A4 dargestellt, die mit der Öffnung nach unten aufgehängt sind. Aus jedem der Infusionsbehälter führt eine Ablaufleitung B1,B2,B3,B4 zu einem gemeinsamen Sammelpunkt 10. Jede der Ablaufleitungen B1 bis B4 enthält eine Tropfkammer C1 bis C4 mit Tropfenzähler, die nachfolgend noch erläutert werden. Im Fließweg hinter der Tropfkammer ist jeweils ein steuerbares Ventil D1 bis D4 in der Ablaufleitung vorgesehen. Jedem Infusionsbehälter A1 bis A4 ist ein dreistelliger Vorwahlschalter S1 bis S4 zugeordnet, an dem die betreffende Infusionsrate in ml/h von Hand eingestellt werden kann. Sämtliche Vorwahlschalter S1 bis S4 sind mit dem Steuergerät 11 verbunden, damit die eingestellten Infusionsraten in das Steuergerät eingegeben werden können. Das Steuergerät 11 empfängt außerdem die Signale der Tropfendetektoren, die den einzelnen Tropfkammern C1 bis C4 zugeordnet sind. Anhand dieser Daten steuert das Steuergerät 11 die Ventile D1 bis D4 in zyklischer Folge so, daß jeweils eines der Ventile geöffnet, während die übrigen Ventile geschlossen sind.

Von dem Sammelpunkt 10 führt eine Sammelleitung 12, die mit einem Katheter o.dgl. verbunden ist, zum Patienten. Die Sammelleitung 12 enthält eine volumetrische Pumpe 13, die über eine Steuerleitung 14 vom Steuergerät 11 gesteuert ist und die in der Sammelleitung 12 enthaltene Flüssigkeit mit der vorgegebenen Zuführrate zum Patienten fördert. Zwischen der Sammelstelle 10 und der Pumpe 13 befindet sich in der Sammelleitung 12 ein Durchflußmesser 15, der im vorliegenden Fall aus einer Tropfkammer 16 mit Tropfendetektor 17 besteht.

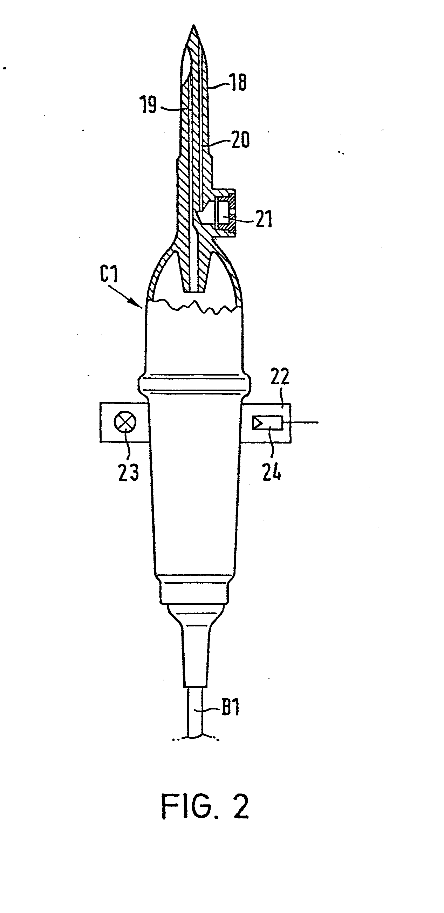

In Fig. 2 ist das Übertragungsgerät dargestellt, dessen Einstechdorn 18 durch den Elastomerstopfen hindurchgestochen wird, mit dem der Infusionsbehälter A1 verschlossen ist. Der Einstechdorn 18 weist einen Flüssigkeitskanal 19 und einen Luftkanal 20 auf, die am vorderen Ende in der Nähe der Spitze austreten. Der Flüssigkeitskanal 19 führt in die Tropfkammer C1 hinein, die an ihrem unteren Ende mit der Ablaufleitung B1 verbunden ist. Am rückwärtigen Ende des Einstechdornes 18 befindet sich ein Luftfilter 21, über welches das rückwärtige Ende des Luftkanals 18 mit der Außenluft in Verbindung steht. Die Tropfkammer C1 besteht im vorliegenden Fall aus lichtdurchläßigem Material. Um die Tropfkammer herum ist der Tropfendetektor 22 angeordnet, der aus einer Lichtquelle 23 und einem Fotosensor 24 besteht. Der Fotosensor 24 erzeugt jedesmal dann ein Signal, wenn ein Flüssigkeitstropfen die aus der Lichtquelle 23 und dem Fotosensor 24 gebildete Lichtschranke durchquert. Anstelle des dargestellten optischen Tropfendetektors können auch andere Tropfendetektoren verwendet werden, die mit Ultraschall, elektrischem Feld, mit Magnetfeld o.dgl. arbeiten oder solche Tropfendetektoren, die auf Druckänderungen ansprechen, z.B. Piezo-Wandler. Wichtig ist nur, daß jeder Flüssigkeitstropfen, der aus der Flüssigkeitsleitung 19 in die Tropfkammer C1 fällt, ein Signal auslöst, das in der noch zu erläuternden Weise weiterverarbeitet wird. Der Flüssigkeitskanal 19 ist so eng, daß die Flüssigkeit durch ihn hindurch tropfenweise in die Tropfkammer C1 fällt. Die Tropfkammern C2 bis C4 sind in gleicher Weise ausgebildet und mit einem Einstechdorn versehen wie die in Fig. 2 dargstellte Tropfkammer C1.

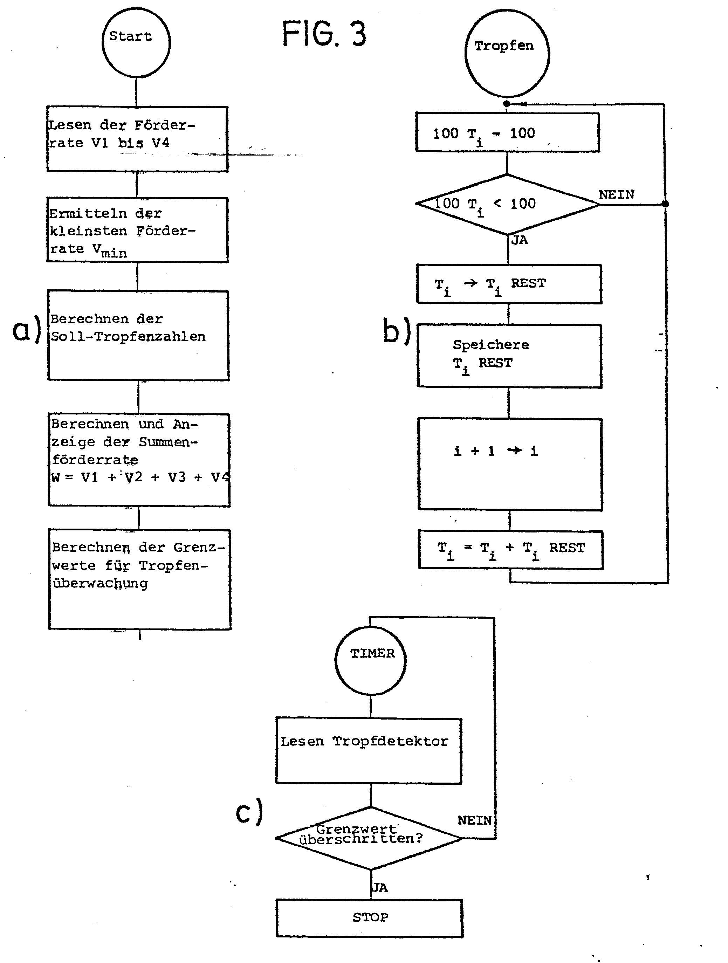

Das Steuergerät 11 ist beispielsweise ein Rechner, der nach den in Fig. 3 dargestellten Flußdiagrammen programmiert ist. Auf das Drücken einer Start/Stop-Taste 25 hin, werden die Förraten V1 bis V4, die an den Vorwahlschaltern S1 bis S4 eingestellt worden sind, in das Steuergerät 11 eingelesen (Fig. 3a). Danach wird aus den Förderraten V1 bis V4 diejenige Förderrate $V_{min}$ ermittelt, die den kleinsten Wert hat. Dann erfolgt die Berechnung der Tropfenzahlen nah der Formel

$$100\ T_i = V_1 \frac{600}{V_{min}}.$$

Hierin ist i die Indexzahl des jeweiligen Kanals, also eine Zahl von 1 bis 4. Die Tropfenzahl ist mit 100 multipliziert, damit der Rechner nur ganze Zahlen (und keine Stellen hinter dem Komma) verarbeiten muß. Die Zahl 600 resultiert daraus, daß die Mindesttropfenzahl, die der kleinsten Förderrate $V_{min}$ entspricht, auf 6 Tropfen festgelegt worden ist. Nach der oben angegebenen Formel werden die Tropfenzahlen für alle vier Kanäle ermittelt. Danach wird die Summenförderrate W = $V_1$ + $V_2$ + $V_3$ + $V_4$ ermittelt, um die Pumpe 13 mit einer Förderrate zu steuern, die dieser Summenförderrate W entspricht. Schließlich werden für jeden Kanal die Grenzwerte der Tropfenüberwachung festgelegt. Dies bedeutet, daß die Minimalzeit und die Maximalzeit definiert wird, in der 25 Tropfen (entsprechend 5ml der Flüssigkeit) fallen müssen.

Der eigentliche Steuervorgang erfolgt nach dem Flußdiagramm der Fig. 3b. Nach Erkennung eines Tropfens durch den Tropfendetektor wird von dem Wert 100 $T_i$ der Betrag von 100 subtrahiert. Wenn die Berechnung der Tropfenzahl $T_2$ für den zweiten Kanal ergeben hat, daß

in jeder Öffnungsperiode des Ventils D2 5,35 Tropfen fallen sollen, dann wird von dem Wert $100\,T_i = 535$ der Wert 100 subtrahiert. Außerdem wird geprüft, ob $100\,T_i$ kleiner ist als 100. Wenn $100\,T_i$ größer oder gleich 100 ist, wird derselbe Vorgang anschließend wiederholt, sobald das Fallen des nächsten Tropfens erkannt worden ist.

Wenn auf diese Weise der ursprüngliche Wert von 535 auf den Wert 35 reduziert worden ist, nachdem 5 Tropfen gefallen sind, ist $100\,T_i$ kleiner als 100 geworden (nämlich 35). Dieser Restwert $T_i$ REST wird gespeichert, um für den nächsten Zyklus zur Verfügung zu stehen. Dann wird der Wert von i um 1 erhöht, d.h. es folgt die Verarbeitung für den nächsten Kanal. Bei dieser Verarbeitung wird der berechneten Tropfenzahl $T_i$ der gespeicherte Restwert $T_i$ REST hinzuaddiert, um den neuen Wert von $T_i$ zu erhalten, mit dem dann die Verarbeitung in Abhängigkeit von dem Fallen der Tropfen wiederholt wird.

Auf die beschriebene Weise wird erreicht, daß bei nicht-ganzzahligen Tropfenzahlen (hier: 5,35 Tropfen) der Restbetrag (hier 0,35) nicht verlorengeht. Vielmehr werden die Restbeträge für denselben Kanal kumulativ aufaddiert. Bei dem gewählten Beispiel bedeutet dies, daß $100\,T_i$ bei dem zweiten Zyklus den Wert 570 (535 + 35) hat und bei dem dritten Zyklus den Wert 605 (535 + 35 + 35). Bei dem dritten Zyklus werden daher nicht 5 Tropfen erzeugt, wie bei den beiden vorhergehenden Zyklen, sondern 6 Tropfen. Der verbleibende Restwert 5 wird gespeichert. Die Tropfenzahl, die in jedem Kanal abgegeben wird, ist daher von Zyklus zu Zyklus nicht immer genau gleich, sondern die Tropfenzahlen können um einen Tropfen voneinander abweichen.

Durch die Berücksichtigung des Restbetrages wird eine sehr genaue Einhaltung der eingestellten Infusionsraten erzielt.

Nach dem Flußdiagramm der Fig. 3c wird die Einhaltung der berechneten Grenzwerte für die Tropfenüberwachung geprüft. Ein Zeitglied (Timer) überwacht die Zeit zwischen einer bestimmten Anzahl von Tropfen (z.B. 25 Tropfen), wenn für das Fallen dieser Tropfen mehr oder weniger Zeit gebraucht wird als die berechneten Grenzwerte angeben, wird ein STOP-Befehl erzeugt. Liegt die Zeit für das Fallen von 25 Tropfen jedoch innerhalb des vorgegebenen Zeitfensters, dann wird der Betrieb der Vorrichtung und die Überwachung fortgesetzt.

Die Summenförderrate $W = V1 + V2 + V3 + V4$ wird an einer Anzeigevorrichtung 26 des Steuergerätes 11 angezeigt. Auf diese Weise kann außer den einzelnen Infusionsraten auch die Gesamt-Infusionsrate abgelesen werden. Die Gesamt-Infusionsrate W wird über Leitung 14 (Fig. 1) der Pumpensteuerung 27 für die Pumpe 13 zugeführt. Die Pumpensteuerung 27 erhält außerdem das Signal des Durchflußmessers 15, das der tatsächlichen Förderrate entspricht. Durch Signalvergleich zwischen Sollwert W und Istwert kann festgestellt werden, ob alle Kanäle ordnungsgemäß arbeiten oder ob einer der Infusionsbehälter A1 bis A4 leer geworden ist oder ob eine andere Störung stattgefunden hat. Ergibt sich eine größere Abweichung zwischen Sollwert und Istwert, dann gibt die Pumpensteuerung 27 ein entsprechendes Alarmsignal an das Steuergerät 11.

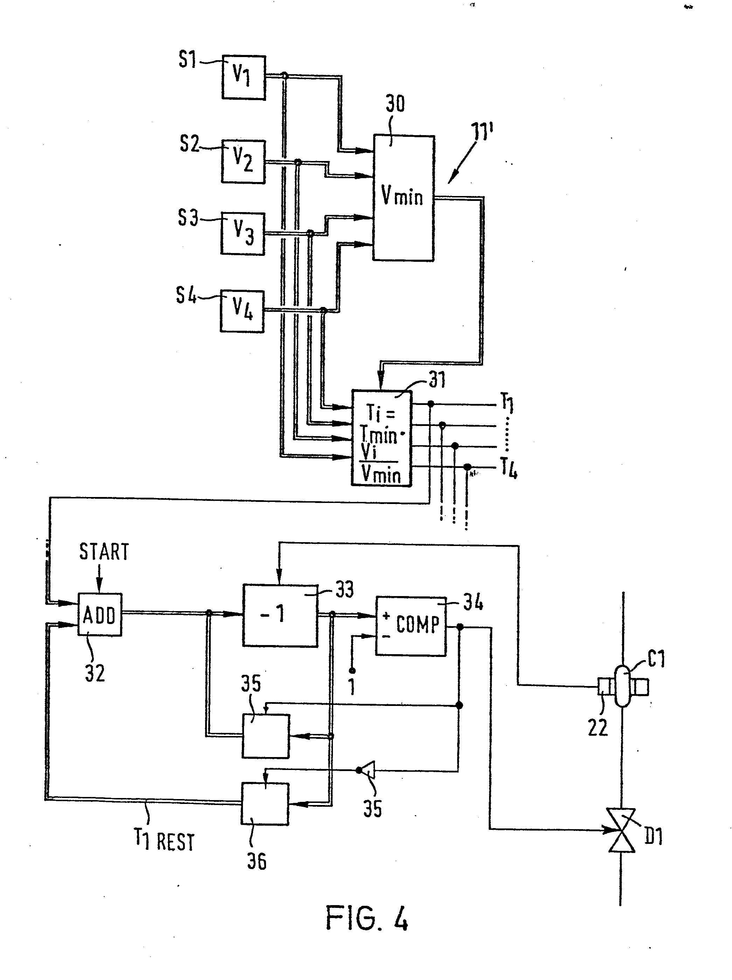

In Fig. 4 ist ein Ausführungsbeispiel dargestellt, bei dem das Steuergerät 11' nicht ein Rechner, sondern eine elektrische Schaltung ist. Die Vorwahlschalter S1 bis S4, an denen die Förderraten V1 bis V4 eingestellt sind, sind an einen Minimalwertdetektor 30 angeschlossen, der den Minimalwert $V_{min}$ ermittelt und einer Rechenschaltung 31 zuführt. Diese Rechenschaltung empfängt außerdem die Werte der eingestellten Infusionsraten V1 bis V4 und bildet aus diesen Werten die Tropfenzahlen nach der Formel

$$T_i = T_{min} \frac{V_i}{V_{min}}.$$

Hierin bezeichnet i jeweils einen der Kanäle von 1 bis 4.

In Fig. 4 ist nur die Weiterverarbeitung der Tropfenzahl $T_1$ dargestellt. Die Tropfenzahlen $T_2$ bis $T_4$ werden in entsprechenden Schaltungen weiterverarbeitet, die aus Gründen der Übersichtlichkeit nicht dargestellt sind. Die Tropfenzahlen $T_i$ werden bei dem Ausführungsbeispiel der Fig. 4 bis auf 2 Stellen hinter dem Komma angegeben. Die Tropfenzahl $T_1$ wird einem Addierer 32 zugeführt. Der Ausgang des Addierers 32 ist mit einer Schaltung 33 verbunden, die von dem zugeführten Wert den Betrag von 1 subtrahiert, wenn von dem Tropfendetektor 22 ein Tropfen festgestellt wird. Der Ausgang der Schaltung 33 ist mit einem Komparator 34 verbunden, dem als Referenzsignal der Wert "1" zugeführt wird. Der Komparator 34 erzeugt ein Ausgangssignal, wenn der an seinem Eingang anstehende Wert mindestens "1,00" ist. Das Ausgangssignal des Komparators 34 öffnet eine Torschaltung 35, die das Ausgangssignal der Schaltung 33 auf den Eingang dieser Schaltung 33 zurückkoppelt. Beim nächstfolgenden Tropfen wird daher das Eingangssignal der Schaltung 33 um den Wert "1,00" verringert.

Das Ausgangssignal des Komparators 34 wird über einen Inverter 35 dem Steueranschluß einer weiteren Torschaltung 36 zugeführt, welche das Rest-Signal $T_1$ REST zu dem zweiten Eingang des Addierers 32 durchläßt, wenn der Wert am Eingang des Komparators 34 kleiner als "1,00" geworden ist. Das Ventil D1 wird durch den Komparator 34 solange geöffnet gehalten, solange der Komparator 34 feststellt, daß die an seinem Eingang anstehende Tropfenzahl größer ist als "1,00". Die Torschaltung 36 enthält ein Register, das denjenigen Restwert, der am Ende eines Zyklus vorhanden war, speichert und ihn beim Beginn des nächsten Zyklus dem Addierer 32 zuführt. Wenn der beschriebene Kanal 1 einen Zyklus beendet hat, wird der Kanal 2 in der gleichen Weise aktiviert, um die gleichen Rechenvorgänge auszuführen.

**A n s p r ü c h e**

1. Vorrichtung zur dosierten gleichzeitigen Infusion
   von Lösungen aus mehreren Infusionsbehältern (A1
   bis A4) mit Ablaufleitungen (B1 bis B4), die von
   den Behältern zu einer Sammelleitung (12) führen und
   jeweils ein gesteuertes Ventil (D1 bis D4) enthalten,
   einer in der Sammelleitung (12) vorgesehenen Pumpe
   (13) mit wählbarem Fördervolumen und mit einem
   Steuergerät (11, 11'), das die Ventile (D1 bis D4)
   entsprechend eingestellten Infusionsraten (V1 bis V4)
   der einzelnen Lösungen zeitlich nacheinander zyklisch
   öffnet,
   d a d u r c h   g e k e n n z e i c h n e t,
   daß die Ablaufleitungen (B1 bis B4) jeweils eine belüftete
   Tropfkammer (C1 bis C4) mit einem Tropfendetektor (22)
   enthalten, daß das Steuergerät (11, 11') die Signale der
   Tropfendetektoren (22) empfängt und jeweils nach einer der
   Infusionsrate entsprechenden Tropfenzahl die Umschaltung auf
   ein anderes Ventil vornimmt, daß das Steuergerät (11,11')
   einen Minimalwertdetektor enthält, der aus den eingegebenen
   Infusionsraten (V1 bis V4) die kleinste Infusionsrate
   ($V_{min}$) ermittelt und dieser kleinsten Infusionsrate eine
   vorgegebene Mindesttropfenzahl zuordnet und daß das Steuergerät eine Recheneinrichtung (31) enthält, die den übrigen
   Infusionsraten die betreffenden Tropfenzahlen proportional
   zu der Mindesttropfenzahl ($T_{min}$) zuordnet.

2. Vorrichtung nach Anspruch 1, dadurch gekennzeichnet daß
   die Recheneinrichtung (31) aus den eingegebenen Infusionsraten (V1 bis V4) proportionale Tropfenzahlen (T1 bis T4)
   errechnet und im Falle einer nicht-ganzzahligen Tropfenzahl einer Lösung eine Abrundung auf die niedrigere ganze Zahl vornimmt, den sich ergebenden Rest ($T_i$ REST) speichert und den Rest bei dem nächstfolgenden Zyklus zu der nichtganzzahligen Tropfenzahl derselben Lösung hinzuaddiert.

3. Vorrichtung nach Anspruch 1 oder 2, dadurch gekennzeichnet, daß die Pumpe (13) mit einer der Summe (W) der Infusionsraten der einzelnen Lösungen entsprechenden Förderrate betrieben ist.

4. Vorrichtung nach einem der Ansprüche 1 bis 3, dadurch gekennzeichnet, daß in der Sammelleitung (12) ein Durchflußmesser (15) vorgesehen ist, der bei Abweichungen von der vorgesehenen Förderrate ein Alarmsignal auslöst.

5. Vorrichtung nach Anspruch 4, dadurch gekennzeichnet, daß der Durchflußmesser (15) aus einer Tropfkammer (16) mit Tropfenzähler (17) besteht.

FIG. 1

0189491

FIG. 2

# FIG. 3

**Start**

Lesen der Förderrate V1 bis V4

Ermitteln der
kleinsten Förderrate $V_{min}$

**a)**

Berechnen der
Soll-Tropfenzahlen

Berechnen und Anzeige der Summenförderrate
$W = V1 + V2 + V3 + V4$

Berechnen der Grenzwerte für Tropfenüberwachung

**Tropfen**

$100\ T_i - 100$

$100\ T_i < 100$ — NEIN

JA

$T_i \rightarrow T_i\ REST$

**b)**

Speichere
$T_i\ REST$

$i + 1 \rightarrow i$

$T_i = T_i + T_i\ REST$

**TIMER**

Lesen Tropfdetektor

**c)**

Grenzwert
überschritten? — NEIN

JA

STOP

FIG. 4

**0189491**

Nummer der Anmeldung

EP   85 10 0793

Europäisches
Patentamt

**EUROPÄISCHER RECHERCHENBERICHT**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A,D | DE-A-2 855 713  (M. DOEHN)<br>* Anspruch 1; Figuren 1, 2 *<br><br>--- | 1 | A 61 M  5/14 |
| A | WO-A-8 203 554  (D. KAMEN)<br>* Seite 5, Zeile 14 - Seite 6, Zeile 21; Figur 2 *<br><br>--- | 1 | |
| A | FR-A-2 258 870  (C. MOULET)<br><br>--- | | |
| A,D | DE-A-2 730 736  (BURRON MEDICAL PRODUCTS INC.)<br><br>----- | | |

| RECHERCHIERTE SACHGEBIETE (Int. Cl.4) |
|---|
| A 61 M   5/00 |

Der vorliegende Recherchenbericht wurde für alle Patentansprüche erstellt

| Recherchenort | Abschlußdatum der Recherche | Prüfer |
|---|---|---|
| BERLIN | 06-09-1985 | MASSALSKI W. |